(19) Europäisches Patentamt
European Patent Office
Office européen des brevets

(11) Publication number: 0 199 506 A2

(12) EUROPEAN PATENT APPLICATION

(21) Application number: 86302695.1

(22) Date of filing: 11.04.86

(51) Int. Cl.[4]: C 07 C 19/02
C 07 C 17/16

(30) Priority: 16.04.85 GB 8509731

(43) Date of publication of application:
29.10.86 Bulletin 86/44

(84) Designated Contracting States:
BE CH DE FR GB IT LI NL

(71) Applicant: The British Petroleum Company p.l.c.
Britannic House Moor Lane
London EC2Y 9BU(GB)

(72) Inventor: Hodgson, Philip Kenneth Gordon
The British Petroleum Company p.l.c. Chertsey Road
Sunbury-on-Thames Middlesex, TW16 7LN(GB)

(72) Inventor: Stewart, Nevin John
The British Petroleum Company p.l.c. Chertsey Road
Sunbury-on-Thames Middlesex, TW16 7LN(GB)

(74) Representative: MacLeod, Malcolm
BP INTERNATIONAL LIMITED Patents Division Chertsey Road
Sunbury-on-Thames Middlesex TW16 7LN(GB)

(54) **Preparation of alkyl halides.**

(57) Alkyl halides are prepared by reacting an alcohol with a halogenating agent in the presence of a compound of a metal of Groups IA or IIA of the Periodic Table as catalyst.

The performance of the catalyst is generally improved by the addition of a solubilising agent.

EP 0 199 506 A2

PREPARATION OF ALKYL HALIDES

This invention relates to a process for the preparation of alkyl halides from organic hydroxyl compounds in which the hydroxyl group is attached to an aliphatic carbon atom.

It is known that alcohols can be converted into corresponding chloro-compounds by reaction with thionyl or sulphuryl chloride via an intermediate chlorosulphite or chlorosulphate ester. However yields of the desired product are low because of competing decomposition reactions.

The provision of soluble chloride ions by the addition of a tertiary amine results in some improvement, but yields are still far from quantitative, particularly in the preparation of secondary alkyl halides.

Furthermore, extended reaction times are often necessary to achieve a satisfactory level of chlorination even when amine catalysis is employed.

We have now discovered that the use of an inorganic catalyst in such a halogenation reaction results in both a shorter reaction period and an increased yield of desired product. Contamination of the product with unwanted by-products is also reduced.

Thus according to the present invention there is provided a process for the production of an alkyl halide of formula (I):

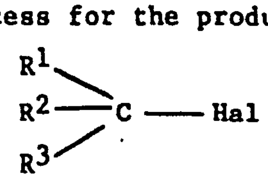

(I)

which process comprises reacting an alcohol of formula (II)

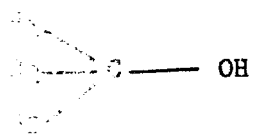 (II)

$R_1$, $R_2$ and $R_3$ which may be the same or different, are ...gen atoms or alkyl groups containing 1 to 24 carbon atoms, ... be substituted by aromatic or other inactive ...uents with a halogenating agent in the presence of a compound ...etal of Groups IA or IIA of the Periodic Table.

...erably $R^1$ and $R^2$ are alkyl groups containing 1 to 12 carbon ... and $R^3$ is a hydrogen atom.

...n certain cases, particularly strongly polar feedstocks, the ...yst may be derived by adding the free metal to the reactants to ... a compound in situ, but more commonly a pre-formed compound ... be used.

Suitable catalysts include the chlorides, hydroxides, alkoxides ...lkanoates of lithium, sodium, potassium, rubidium, caesium, ...nesium, calcium, strontium and barium.

The performance of the catalyst is generally improved by the ...tion of a solubilising agent. Suitable agents include crown ...ers, polyalkoxy compounds containing three or more alkoxy groups ... molecule, such as dimethyl polyethylene glycol, and dipolar, ...tic solvents, such as dimethyl sulphoxide.

Preferred halogenating agents are chlorinating agents such as ...l chloride and sulphuryl chloride.

...e reaction is suitably effected at a temperature in the range ...120°C, preferably in the range 75° to 85°C.

Pressure is not a significant parameter and the reaction is ...ore most conveniently carried out at ambient pressure.

...he molar ratio of alcohol to halogenating agent is suitably in ...nge 1:1 to 1:5, preferably 1:1 to 1:1.5.

...e quantity of catalyst employed is preferably in the range ...% by weight, expressed as a percentage by weight of the ...

...e reaction may be effected in the presence or, preferably, ...ence of a solvent. Suitable solvents include



BAD ORIGINAL

1,2-dichloroethane, toluene and chloroform.

The invention is illustrated with reference to the following Examples. Example 1 is not in accordance with the invention and is provided for comparison.

Example 1

1.3g (0.01M) octan-2-ol was heated with with stirring at 80°C with 1.43g (0..12M) thionyl chloride. The product was isolated by pumping in vacuo.

After 4.5 hours $^{13}C$ NMR (Nuclear Magnetic Resonance) spectroscopy indicated the production of 31% by wt octyl chloride, 34% chlorosulphite, 11% oct-1-ene, 10% cis-oct-2-ene and 14% trans-oct-2-ene.

Example 2

3.0g (0.03M) octan-2-ol was heated with stirring at 80°C with 4.29g (0.036M) thionyl chloride and 39 mg (1% by wt) lithium hydroxide monohydrate (39 mg, 1%). The product was isolated as before.

After 3 hours $^{13}C$ NMR spectroscopy indicated the production of 43% by wt octyl chloride, 4% chlorosulphite, 11% oct-1-ene, 16% cis-oct-2-ene, 16% trans-oct-2-ene and 10% other material.

Example 3

3.9g (0.03M) octan-2-ol was heated with stirring at 80°C with 4.29g (0.036M) thionyl chloride, 39 mg (1% by wt) lithium hydroxide monohydrate and 160 mg 12-crown-4-ether. The product was isolated as before.

After 0.5 hours $^{13}C$ NMR spectroscopy indicated an octyl chloride content of 75% by wt. No remaining chlorosulphite could be detected and other product levels were 2% oct-1-ene, 3.5% cis-oct-2-ene, 5.5% trans-oct-2-ene and 14% other material.

A comparison of Examples 1 and 2 shows that using a lithium hydroxide catalyst increases both the reaction rate and the yield of desired product.

Example 3 shows that these effects are greatly enhanced by the addition of 12-crown-4-ether as a solubilising agent for the catalyst.



BAD ORIGINAL

Claims:

1. A process for the production of an alkyl halide of formula (I):

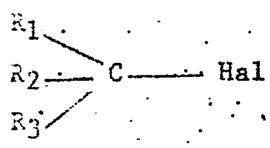

(I)

which process comprises reacting an alcohol of formula (II):

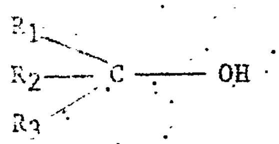

(II)

wherein $R^1$, $R^2$ and $R^3$ which may be the same or different are hydrogen atoms or alkyl groups containing 1 to 24 carbon atoms, which may be substituted by aromatic or other inactive substituents with a halogenating agent,
characterised by the fact that the reaction is carried out in the presence of a compound of a metal of Groups IA or IIA of the Periodic Table as a catalyst.

2. A process according to claim 1 wherein $R^1$ and $R^2$ are alkyl groups containing 1 to 12 carbon atoms and $R^3$ is a hydrogen atom.

3. A process according to claim 1 wherein the catalyst is selected from the group consisting of the chlorides, hydroxides, alkoxides and alkanoates of lithium, sodium, potassium, rubidium, caesium, magnesium, calcium, strontium and barium.

4. A process according to any of the preceding claims wherein a solubilising agent is added to the catalyst.

5. A process according to claim 4 wherein the solubilising agent is a crown ether, a polyalkoxy compound containing three or more alkoxy groups per molecule or a dipolar aprotic solvent.



BAD ORIGINAL

6. A process according to any of the preceding claims wherein the halogenating agent is thionyl chloride or sulphuryl chloride.

7. A process according to any of the preceding claims wherein the halogenation reaction effected at a temperature in the range 30° to 120°C.

8. A process according to any of the preceding claims wherein the molar ratio of alcohol to halogenating agent is in the range 1:1 to 1:5.

9. A process according to any of the preceding claims wherein the quantity of catalyst employed is in the range 0.1% to 5% by weight, expressed as a percentage by weight of the alcohol.